# EUROPÄISCHE PATENTSCHRIFT

(11) **EP 0 925 037 B1**
(45) Veröffentlichungstag und Bekanntmachung des Hinweises auf die Patenterteilung: **17.12.2003**
(21) Anmeldenummer: 98941396.8
(22) Anmeldetag: 21.07.1998
(51) Int. Cl.: A61B 17/28

(54) **VORRICHTUNG ZUM BEGRENZEN DER KRAFTÜBERTRAGUNG AN CHIRURGISCHEN INSTRUMENTEN**
DEVICE FOR LIMITING THE FORCE TRANSMITTED TO SURGICAL INSTRUMENTS
DISPOSITIF DESTINE A LIMITER LA TRANSMISSION DE FORCE SUR DES INSTRUMENTS CHIRURGICAUX

(30) Priorität: 22.07.1997 DE 19731453
(43) Veröffentlichungstag der Anmeldung: 30.06.1999
(73) Patentinhaber: Karl Storz GmbH & Co. KG, 78532 Tuttlingen (DE)
(72) Erfinder: BOCHE, Hartmut, D-88090 Immenstaad (DE)
(74) Vertreter: Weller, Wolfgang, Dr.rer.nat.
(86) Internationale Anmeldenummer: EP9804577
(87) Internationale Veröffentlichungsnummer: WO99004703

(56) Entgegenhaltungen:
- EP-A- 0 674 878
- DE-A- 3 819 123
- DE-A- 4 131 176
- US-A- 5 573 530

## Beschreibung

Die Erfindung betrifft eine Vorrichtung zum Begrenzen der Kraftübertragung von einem bewegbaren Betätigungselement eines chirurgischen Instrumentes auf die durch das Betätigungselement bewegten arbeitenden Teile, insbesondere Maulteile von chirurgischen Zangen, mit einem Betätigungselement, das ein Gehäuse und zumindest eine relativ zum Gehäuse beweg bare dem Betätigungs element zungehörige, quer zu dessen ßewegungsrichtung austeigende Keilfläche aufweist. Die Erfindung betrifft ferner chirurgische Instrumente, die mit einer derartigen Vorrichtung versehen sind.

Eine derartige Vorrichtung ist aus der DE 41 31 176 A bekannt. Bei dieser Vorrichtung erfolgt im Überlastfall durch eine Spreizung eines Teiles der Betätigungsstange ein formschlüssiger Eingriff in einen ortsfesten Teil des Zangengehäuses, z.B. durch ein elastisches Rohrstück.

Derartige chirurgische Instrumente können Faß-, Halte- und Präparierzangen, Scheren oder sonstige Instrumente sein, bei denen durch Handkraft über ein hin- und herbewegbares Betätigungselement arbeitende Teile, meist Maulteile, bewegt werden.

Faß-, Halte- und Präparierzangen sind z.B. aus dem Katalog "Endoskopische Chirurgie, 2. Ausgabe, 1/94, Abschnitt 4, Präparier- und Faßzangen" der Karl Storz GmbH & Co., Tuttlingen, Deutschland, bekannt.

Diese bekannten Zangen weisen einen langerstreckten zylindrischen Schaft auf, an dessen distalem Ende zumindest zwei Maulteile angeordnet sind. Am proximalen Ende des Schaftes ist ein Griff angeordnet, der die Form zweier scherenartiger Griffteile aufweist. Eines der Griffteile ist fest mit dem Schaft verbunden, das andere, das sogenannte bewegliche Griffteil, ist über ein Scharnier mit dem unbeweglichen Griffteil verbunden. Ein Mechanismus, der zum Öffnen und Schließen der Maulteile dient, weist ein durch den Schaft hindurchgehendes stabförmiges Betätigungselement auf, das am distalen Ende mit den Maulteilen verbunden ist. Am proximalen Ende reicht das Betätigungselement über das Ende des feststehenden Griffteiles hinaus und ist gelenkig, meist über ein Kugelpfannengelenk mit dem oberen äußeren Ende des beweglichen Griffteiles verbunden. Ein Verschwenken des beweglichen Griffteiles um die Scharnierachse relativ zum feststehenden Griffteil bewirkt ein lineares Hin- und Herverschieben des Betätigungselementes im Schaft. Diese lineare bzw. axiale Hin- und Herbewegung wird in eine Schwenkbewegung der Maulteile umgesetzt.

Derartige Zangen finden bei der weit verbreiteten minimal-invasiven Chirurgie Einsatz. Die Zangen werden dabei über Trokare in den Körper eingeführt.

Durch die bei der minimal-invasiven Chirurgie notwendige Miniaturisierung der Maulteile sind diese empfindlich für Belastungen, da bei gleichen Handkräften die durch die Miniaturisierung ertragbaren Kräfte kleiner werden. Dies gilt auch bei normalgroßen dynamischen Geräten, wenn der Auslegungsfall überschritten wird. Das bewegliche Griffelement ist als Hebel ausgebildet, wobei die Hebelachse die Scharnierachse darstellt, an der dieses bewegliche Griffelement am anderen Griffelement angelenkt ist. Der Abstand von der Scharnierachse zu der Stelle, an der das bewegliche Griffelement mit dem zu verschiebenden Betätigungselement verbunden ist, ist wesentlich kürzer als der Abstand von der Scharnierachse zu der am äußeren Ende des Griffelementes angeordneten Fingeröse. Das Übersetzungsverhältnis beträgt etwa 10:1, das heißt, die Schließkraft einer Hand, in etwa 10 kp, wird durch die Hebelwirkung auf das Zehnfache verstärkt, also auf etwa 100 kp. Eine Kraftbegrenzung ist bei den ausgebildeten Kinematiken der bekannten Zangen nicht vorhanden.

Im praktischen Einsatz von chirurgischen Faß- und Haltezangen wurde festgestellt, daß bei der Handhabung versucht wird, einen zu haltenden Gegenstand, beispielsweise einen Kugeltupfer oder eine Nadel, möglichst fest in die Faßzange einzuspannen. Bei schneidenden und insbesondere bei durchschneidenden Maulteilen kann die Gefahr bestehen, falls anstatt eines einfach zu durchtrennenden weichen Gewebeteiles ein Knorpel oder ein Knochenstück zwischen die Maulteile gelangt, daß vom Operateur eine

Schließkraft aufgewandt wird, die über-die sicher ertragbare Kraft der Maulteile hinausgeht. Kräftige Personen können eine Schließkraft von 15 kp oder mehr ausüben, die dann über den Hebel auf das Zehnfache verstärkt wird. Bei häufiger Überbelastung durch Ausüben zu großer Handkräfte und damit zu hoher Belastung der Maulteile kann dies zu Materialermüdung im Maulteilbereich bis hin zu Brüchen der Maulteile führen. Dabei besteht die Gefahr, daß die erwähnten Brüche der Maulteile durch Überlastung während der Operation erfolgen. Dies kann zu unkontrollierten Beschädigungen des Gewebes und der Organe im Operationsbereich führen.

Aus der DE 38 19 123 A1 ist ein chirurgisches Instrument bekannt, das mit einem Überlastschutz versehen ist. Der Überlastschutz besteht aus einem Kraftspeicher, welcher den erhöhten Zug auffängt. Im einfachsten Fall besteht der Kraftspeicher aus einer Schraubenfeder.

Aus der DE 94 07 081 U1 ist eine Überlastsicherung für motorisch betriebene chirurgische Instrumente bekannt, die als Klauenkupplung, Rutschkupplung, Magnetkupplung oder Sollbruchstelle ausgebildet sein kann.

Vor diesem Hintergrund ist es Aufgabe der vorliegenden Erfindung, eine Vorrichtung zu schaffen, durch die Überbelastungen an den arbeitenden Teilen eines chirurgischen Instrumentes sicher verhindert werden können.

Erfindungsgemäß wird die Aufgabe durch eine Vorrichtung gelöst, die dadurch gekennzeichnet ist daß
das Gehäuse durch Bewegen der Keilfläche relativ zum Gehäuse spreizbar ist, und mit einem das Gehäuse in dessen Spreizbereich umfänglich umschließenden, durch den Spreizbereich elastisch spreizbaren Ring.

Das spreizbare Gehäuse mit dem dieses umschließenden elastisch spreizbaren Ring sind so ausgelegt, daß eine bestimmte Kraft notwendig ist, um überhaupt eine bestimmte Spreizung zu erzielen. Diese Kraft entspricht der Kraft, mit der die Maulteile maximal beaufschlagt werden sollen. Werden nun auf das Betätigungselement in einer Richtung Kräfte ausgeübt, die diese maximale Kraft überschreiten, bewirken diese Kräfte eine Spreizung des Gehäuses. Diese Kräfte werden vom Betätigungselement über die Keilfläche auf das Gehäuse abgeleitet und zur reversiblen Verformung (Spreizung) herangezogen. Es findet also eine Relativbewegung zwischen Gehäuse und Keilfläche statt.

Das Ausmaß, in dem das Gehäuse von der Keilfläche gespreizt wird, wird durch den das Gehäuse in dessen Spreizbereich umschließenden spreizbaren Ring begrenzt. Die geometrische Ausgestaltung und die Auswahl des Materials von Gehäuse und spreizbarem Ring im Zusammenhang mit der Neigung der Keilfläche definieren den Lastanstieg über dem Weg für die Vorrichtung zur Kraftbegrenzung. Diese Auswahl an Parametern erlaubt es, bei äußerst schlanker Bauweise der Vorrichtung hohe Kräfte bei Überschreiten eines Schwellwertes in Verformung abzuleiten. Wirken die Kräfte nicht mehr im Übermaß ein, verursacht der um das Gehäuse gelegte elastische spreizbare Ring wieder ein Schließen des Gehäuses, wodurch die Keilfläche samt dem Betätigungselement wieder zurückverschoben wird. Die Vorrichtung arbeitet somit nicht nur als ein dynamischer Kraftbegrenzer, sondern aufgrund der Elastizität von Gehäuse und dieses im Spreizbereich umschließenden Ring als reversibel arbeitende Vorrichtung, so daß nach einem Überlastfall die Vorrichtung wieder in eine Ausgangsstellung zurückgekehrt bzw. umgeformt wird, in der diese für den nächsten Überlastfall bereit ist.

Eine Überlastung der Maulteile wird also dadurch verhindert, daß Kräfte, die über den Auslegungslastfall der Maulteile hinausgehen, von der Vorrichtung dynamisch umgesetzt werden, also nicht auf die Maulteile weitergeleitet werden. Die Maulteile werden somit ausschließlich mit Kräften in einer Höhe belastet, für die sie konzipiert und konstruiert sind, so daß Materialermüdungen, die zu Brüchen führen können, weitgehend ausgeschlossen werden.

Eine solche Vorrichtung zum Begrenzen der Kraftübertragung auf die Maulteile eines chirurgischen Instruments hat ferner den Vorteil, daß der Operateur nicht selbst darauf achten muß, daß er nicht zu viel Kraft auf den Handgriff des Instrumentes ausübt, sondern er sich ganz auf die Vorgänge im Operationsbereich konzentrieren kann.

Somit wird die Aufgabe vollkommen gelöst.

In einer bevorzugten Ausgestaltung der Erfindung ist das Betätigungselement axial längs eines Schaftes des Instrumentes verschiebbar, und die Keilfläche erstreckt sich in axialer Richtung des Betätigungselementes.

Diese Ausgestaltung hat den Vorteil, daß die Vorrichtung an axial verschiebbaren Betätigungselementen, die bei dem überwiegenden Anteil der verwendeten chirurgischen Instrumente verwirklicht sind, einfach integriert werden kann. Die erfindungsgemäß vorgesehene Keilfläche erstreckt sich in axialer Richtung und steigt in radialer Richtung gesehen an, so daß die axiale Bewegung des Betätigungselementes in ein radiales Spreizen des Gehäuses umgesetzt wird. Dies ist konstruktiv einfach umzusetzen, indem zum Beispiel an das Betätigungselement ein oder mehrere Keile angesetzt werden.

In einer weiteren Ausgestaltung der Erfindung liegt der Steigungswinkel α der Keilfläche im Bereich von ca. 8° bis 12°.

Diese Maßnahme hat den Vorteil, daß die relative Bewegung der Keilfläche zum Gehäuse durch die geringe Steigung der Keilfläche harmonisch verläuft und nur ein geringes Spreizen des Gehäuses in radialer Richtung erfolgt, so daß keine bemerkenswerte Durchmessererweiterung erfolgt. Dadurch ist die Vorrichtung auch bei sehr schlank gebauten Instrumenten einsetzbar. Dieser Keilwinkelbereich erlaubt ein klemmfreies

Bewegen der Keilfläche längs der zu spreizenden Gehäuseinnenseite, und zwar sowohl beim Spreizen als auch beim Rückkehren des Keiles beim Schließen, wenn keine Überlastkräfte mehr einwirken. Eine entsprechende Auswahl der Materialien von Gehäuse und darum gelegtem Ring im Zusammenhang mit diesem Keilflächenwinkelbereich erlaubt eine kompakte Bauweise, das heißt, axiale Verschiebebewegungen der Keilfläche finden im Bereich von 1 bis 3 mm statt, die dadurch verursachten radialen Spreizbewegungen liegen im Bereich von Bruchteilen von Millimetern. Dabei ist gleichzeitig sichergestellt, daß auch extrem hohe Überlastkräfte bei übermäßigem Zupacken abgeleitet werden.

In einer weiteren Ausgestaltung der Erfindung ist die Keilfläche als Außenfläche eines Konus ausgebildet.

Hierbei ist vorteilhaft, daß durch den Konus die Kräfte umfänglich gleichmäßig verteilt auf das Gehäuse abgeleitet werden.

In einer weiteren Ausgestaltung der Erfindung weist das Gehäuse sich in Bewegungsrichtung des Betätigungselementes erstreckende, die Gehäusewand durchbrechende Nuten auf.

Durch die Nuten wird das Gehäuse umfänglich segmentiert. Dabei ist von Vorteil, daß die reversible Spreizbarkeit des Gehäuses auf einfache Art und Weise gewährleistet wird, und zwar auch dann, wenn das Gehäuse selbst aus einem wenig deformierbaren Material besteht. Die Enden der Nut bestimmen die Schwenkpunkte, um die die Segmente radial nach außen beim Spreizen verschwenkt werden.

In einer weiteren Ausgestaltung der Erfindung endet jede Nut in einer abgerundeten Aufweitung.

Diese Maßnahme hat den Vorteil, daß die während des Spreizensder umfänglich zwischen den Nuten gelegenen Gehäusewandsegmente auf das Material im Bereich des Endes der Nuten einwirkenden Kerbkräfte reduziert und somit die Spannungskonzentrationen in den Bereichen minimiert werden, die beim Spreizen und Schließen immer wieder großen Belastungen ausgesetzt sind. So werden Materialermüdungen und damit auch Risse oder Kerbbrüche der Gehäusewand auch nach zahlreichen Spreiz- und Schließvorgängen vermieden.

In einer bevorzugten Ausgestaltung der Erfindung weist das Gehäuse vier gleichmäßig um dessen Umfang verteilte Nuten auf.

Dies hat den Vorteil, daß vier Nuten fertigungstechnisch mit vertretbarem Aufwand gefertigt werden können und bei schlanken Instrumenten eine ausreichende Segmentierung vorhanden ist, so daß das Gehäuse aus hochfestem Material, beispielsweise Medizinstahl, hergestellt werden kann. Es versteht sich, daß auch eine größere Anzahl von Nuten vorgesehen werden kann, beispielsweise sechs oder acht Nuten.

In einer weiteren Ausgestaltung der Erfindung weist das Gehäuse im Bereich der Aufweitung umfänglich eine rinnenförmige Vertiefung auf, durch die die Dicke der Wand des Gehäuses reduziert wird.

Diese Maßnahme hat den Vorteil, daß im Bereich der rinnenförmigen Vertiefung die Gehäusewand relativ dünn ist, wodurch die Spreiz- und Schließbewegungen der Gehäusesegmente über die Art eines Filmscharnieres ermöglicht wird.

In einer weiteren Ausgestaltung der Erfindung ist das Gehäuse aus Kunststoff hergestellt.

Hierbei ist von Vorteil, daß das Gehäuse kostengünstig hergestellt und als Austauschteil konzipiert werden kann. Unterschiedliche Kunststoffe können dazu eingesetzt werden, um die Deformationseigenschaft des Gehäuses und damit auch den Lastanstieg über dem Weg für die erfindungsgemäße Vorrichtung zu definieren.

In einer bevorzugten Ausgestaltung weist der elastisch spreizbare Ring einen Schlitz auf.

Diese Maßnahme hat den Vorteil, daß bei an sich sehr festen Materialien die Spreizbarkeit des Ringes definiert und vorbestimmt erfolgen kann. Durch die Geometrie und Auswahl der Materialien des Ringes kann schon eine geringe Spreizbewegung sehr hohe Kräfte notwendig machen, so daß bei besonders schlanken Instrumenten keine merkliche radiale Aufweitung erfolgt. In einem solchen geringen Aufweitungsbereich liegen ideale lineare Federkennlinien-Bedingungen vor, so daß exakt vorausbestimmbare Arbeitsbedingungen des dynamischen Kraftbegrenzers vorhanden sind.

In einer weiteren Ausgestaltung der Erfindung ist der Ring an unterschiedlichen axialen Positionen auf dem Gehäuse anbringbar.

Diese Maßnahme erhöht weiter die Variabilität der Vorrichtung, so daß allein durch die axiale Anlegestellung des Ringes bei an sich sonst gleichen Bauteilen auf unterschiedliche Überlastfälle eingestellt werden kann.

In einer weiteren Ausgestaltung ist der Ring in einer Ringnut am Gehäuse aufgenommen.

Diese Maßnahme hat den Vorteil, daß der Ring einfach montiert und unverlierbar am Gehäuse gehalten ist.

In einer weiteren Ausgestaltung der Erfindung ist der Ring aus metallischen Materialien, insbesondere aus Stahl oder aus Kunststoffmaterial, insbesondere aus faserverstärkten Kunststoffen, hergestellt.

Diese Maßnahme hat den Vorteil, daß zum einen für den medizinischen Betrieb geeignete Materialien herangezogen werden, die in puncto Reinigungs- und Sterilisierungsmöglichkeit geeignet sind und zugleich die elastischen Eigenschaften aufweisen, die eine definierte Spreiz- und Schließbewegung des Gehäuses sicherstellen. Als faserverstärkte Kunststoffe können beispielsweise Carbon-Epoxy, Kevlar-Epoxy, Glas-Epoxy oder ähnliche Kunststoffe vorgesehen sein.

In einer weiteren Ausgestaltung der Erfindung weist das Betätigungselement einen ersten Abschnitt auf, der mit dem Gehäuse fest verbunden ist und weist ferner einen zweiten Abschnitt auf, der vom ersten Abschnitt getrennt ist, wobei der zweite Abschnitt die Keilfläche trägt, die innerhalb des Gehäuses angeordnet ist.

Diese Maßnahme hat den Vorteil, daß im Überlastfall der zweite Abschnitt, der die Keilfläche trägt, die Relativbewegung zwischen Betätigungselement und Gehäuse zur Verursachung der Spreizbewegung verursacht, wobei der erste Abschnitt, der ja fest mit dem Gehäuse verbunden ist, unverändert bleibt. Dadurch ist es möglich, beispielsweise die mit dem ersten Abschnitt fest verbundenen Maulteile in einer definierten Relativendstellung zueinander zu halten und auch im Überlastfall, also bei der Spreizung, nicht weiter zu bewegen. Dies ist besonders dann von Vorteil, wenn in dieser Endstellung schon die Maulteile unter einem Preß- oder Schließdruck aneinanderliegen und im Überlastfall nicht mehr relativ zueinander bewegt werden sollen.

Im zulässigen Lastbereich besteht Kraftschluß zwischen den beiden Abschnitten des Betätigungselementes, so daß beispielsweise die Zange mit Kraftbegrenzungs-Vorrichtung das gleiche Wirkverhalten wie eine Zange ohne Kraftbegrenzung aufweist.
Im Überlastfall wird der Kraftschluß derart unterbrochen, daß die maximal zugelassene Last weiter über das Betätigungselement übertragen, die Überlast jedoch auf andere Bauelemente abgeleitet wird. Die besondere Eigenschaft des erfindungsgemäßen Kraftbegrenzers besteht darin, daß nach Beendigung des Überlastfalls durch selbsttätige Rückstellung der uneingeschränkte Kraftschluß zwischen beiden Abschnitten wiederhergestellt wird und beispielsweise die Zange wieder uneingeschränkt gebrauchsfähig ist. Der Kraftbegrenzer wirkt damit als "dynamischer Kraftbegrenzer", der auf individuelle Überlastfälle in der Anwendung reagiert, ohne die Funktionsfähigkeit des chirurgischen Instruments zu beeinträchtigen.

In einer weiteren Ausgestaltung der Erfindung liegt in einer Normallast-Betriebsstellung der erste Abschnitt am zweiten Abschnitt an, und in der Überlast-Betriebsstellung hat sich der zweite Abschnitt vom ersten Abschnitt gelöst.

Durch das Anliegen der beiden Abschnitte aneinander können hinund hergerichtete Bewegungen über das Betätigungselement spielfrei übertragen werden, erst im Überlastfall löst sich der zweite Abschnitt vom ersten Abschnitt.

Der Schwellwert entspricht der Kraft, die erforderlich ist, daß der uneingeschränkte Kraftschluß zwischen den beiden Abschnitten des Betätigungselementes gerade nicht mehr besteht und daß mittels der Vorrichtung begonnen wird, Kraftanteile auf andere Bauelemente der Vorrichtung abzuleiten. Dies geschieht dadurch, daß die auf das Betätigungselement in einer Richtung ausgeübten Kräfte, die diesen Schwellwert überschreiten, eine Spreizung des Gehäuses bewirken.

In einer weiteren Ausgestaltung der Erfindung steht der erste Abschnitt des Betätigungselementes über eine Schraubverbindung fest mit dem Gehäuse in Verbindung, wobei die Einschraubtiefe eine Einstellung eines Auslegungspunktes der Vorrichtung erlaubt.

Diese Maßnahme hat nun den erheblichen Vorteil, daß bei der Montage ein bestimmter Auslegungspunkt eingestellt werden kann. Beim Einschrauben trifft das Ende des ersten Abschnittes auf das entsprechend gegenüberliegende Ende des zweiten Abschnittes, das die Keilfläche bzw. den Konus trägt. Ein weiteres Eindrehen in das Gehäuse verursacht dann schon ein gewisses Verschieben der Keilfläche, die dann schon in Richtung Spreizen verschoben wird, wobei diesem Spreizen die Gegenkraft durch den äußeren Ring entgegengestellt wird. Erst eine Kraft, die nun ausreichend ist, um die Keilfläche weiterzubewegen, verursacht ein Spreizen des Gehäuses, also eine Bewegung über den Auslegungspunkt hinaus. Dadurch kann an ein und demselben Instrument, das beispielsweise mit unterschiedlichen Maulteilen betrieben wird, ein für die Anwendung optimaler Auslegungspunkt eingestellt werden.

In einer weiteren Ausgestaltung der Erfindung ist das Gehäuse fest mit dem Griff verbunden, wobei das Betätigungselement die Keilfläche trägt, die innerhalb des Gehäuses angeordnet ist.

Diese Maßnahme hat den Vorteil, daß über das durchgehende Betätigungselement immer ein direkter Kraftschluß zwischen den zu bewegenden Maulteilen und dem Griff besteht, was bspw. bei Scheren erforderlich ist, wobei eine Erhöhung der Handkraft am Griff immer in einen zusätzlichen Weg und, abhängig von der Auslegung und/oder Voreinstellung der Vorrichtung, in zunehmende Spreizung von Gehäuse und Ring umgesetzt wird. Die Kraftbegrenzung erfolgt dadurch, daß die Spreizung bei maximal aufbringbaren Handkräften durch die Materialeigenschaften und Bauweisen von Gehäuse und Ring limitiert ist.

Bei einem chirurgischen Instrument, das mit einer erfindungsgemäßen Vorrichtung ausgestattet ist, ist bevorzugt, daß die Vorrichtung im proximalen Bereich des Handgriffes angeordnet ist.

Diese Maßnahme hat den Vorteil, daß die Vorrichtung integriert und somit auch geschützt im Bereich des Handgriffes am proximalen Ende des Schaftes angeordnet werden kann, so daß im Bereich des Schaftes und der Maulteile als solche überhaupt keine Durchmesservergrößerung aufgrund des zusätzlichen Einbaus der Vorrichtung resultiert.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert.

Es zeigen:
- Fig. 1: eine Seitenansicht eines chirurgischen Instrumentes in Form einer Zange, bei der eine erfindungsgemäße Vorrichtung zum Begrenzen der Kraftübertragung eingebaut ist,
- Fig. 2: in stark vergrößertem Maßstab eine Explosionsdarstellung der in Fig. 1 gezeigten Vorrichtung,
- Fig. 3: einen Schnitt längs der Linie III-III in Fig. 2,
- Fig. 4: einen teilweisen Längsschnitt durch die Vorrichtung in zusammengebautem Zustand, und zwar in einer ersten Betriebsstellung,
- Fig. 5: eine der Darstellung von Fig. 4 vergleichbare Darstellung in einer zweiten Betriebsstellung im Überlastfall, und
- Fig. 6: ein prinzipielles Kraft-Weg-Diagramm der erfindungsgemäßen Vorrichtung.

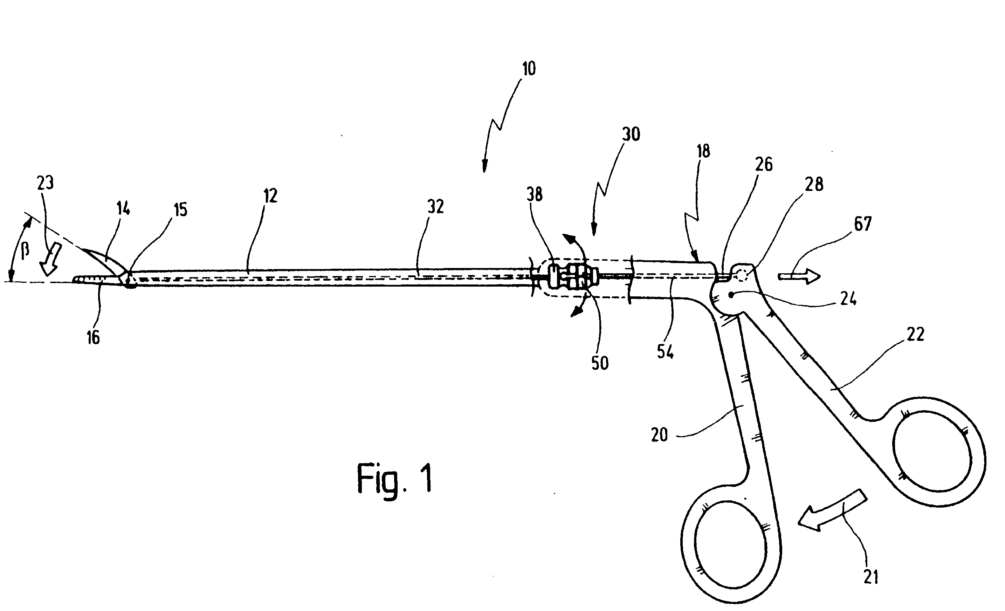

In Fig. 1 ist ein chirurgisches Instrument in Form einer Faßund Präparierzange dargestellt, die in ihrer Gesamtheit mit der Bezugsziffer 10 versehen ist.

Die Zange 10 weist einen langerstreckten rohrförmigen Schaft 12 auf, an dessen distalem Ende zwei Maulteile 14 und 16 angeordnet sind.

Das Maulteil 16 ist starr mit dem Schaft 12 verbunden, das Maulteil 14 ist um eine Achse 15 verschwenkbar angeordnet.

Am proximalen Ende des Schaftes 12 ist ein Griff 18 angeordnet.

Der Griff 18 weist ein feststehendes Griffelement 20 auf, das fest mit dem Schaft 12 verbunden ist. Ein bewegliches Griffelement 22 ist um eine Scharnierachse 24 schwenkbar am feststehenden Griffelement 20 angeordnet.

Durch den Schaft 12 hindurchgehend ist ein stabförmiges Betätigungselement 26 angeordnet, das am distalen Ende mit dem beweglichen Maulteil 14 verbunden ist, und zwar etwas im Abstand zu dessen Schwenkachse 15. Am proximalen Ende reicht das Betätigungselement 26 über das feststehende Griffelement 20 hinaus und ist in einem Kugelpfannengelenk 28 des beweglichen Griffelementes 22 aufgenommen. In Fig. 1 sind die Maulteile 14 und 16 geöffnet, sie schließen somit den Öffnungswinkel β ein. Der maximale Öffnungswinkel beträgt üblicherweise 50°.

Wird die Zange 10 in dieser Stellung ergriffen, wobei zwei Finger einer Hand in die endseitigen Fingerösen der Griffelemente 20 bzw. 22 eingreifen, und wird das bewegliche Griffelement 22 auf das feststehende Griffelement 20 hin bewegt, wie das in Fig. 1 durch einen Pfeil 21 angedeutet ist, wird das Betätigungselement 26 nach proximal verschoben, wie das durch einen Pfeil 67 angedeutet ist. Diese lineare Verschiebebewegung des Betätigungselementes 26 wird in eine Schließbewegung der Maulteile umgesetzt, das heißt, das bewegliche Maulteil 14 wird auf das unbewegliche Maulteil 16 hin verschwenkt, wie das in Fig. 1 durch einen Pfeil 23 angedeutet ist.

Aus Fig. 1 ist auch zu erkennen, daß der Abstand von der Fingeröse zur Scharnierachse 24 wesentlich größer ist als der Abstand von der Scharnierachse 24 zum Kugelpfannengelenk 28. Somit wird die Kraft, die durch einen in die Fingeröse eingeschobenen Finger auf das bewegliche Griffelement 22 einwirkt, über die Hebelwirkung verstärkt, übliche Übersetzungsverhältnisse betragen 10:1. Beträgt die Kraft, die auf das bewegliche Griffelement 22 einwirkt, etwa 10 kp, wird diese um den Faktor 10, also auf 100 kp, verstärkt. Für solche Kräfte sind üblicherweise derartige chirurgische Instrumente ausgelegt. Es können aber von der Kraft einer Hand wesentlich höhere Kräfte ausgeübt werden, die im Bereich von 15 kp oder mehr liegen, die dann entsprechend der Hebelwirkung auf das Zehnfache verstärkt werden.

Um solche übermäßigen Kräfte nicht auf die Maulteile 14 und 16 abzuleiten, ist eine Vorrichtung 30 zum Begrenzen der Kraftübertragung vorgesehen.

Wie aus Fig. 1 zu erkennen ist, ist die Vorrichtung am distalen Ende des feststehenden Griffelementes 20 angeordnet, von dessen Ende der Schaft 12 vorspringt.

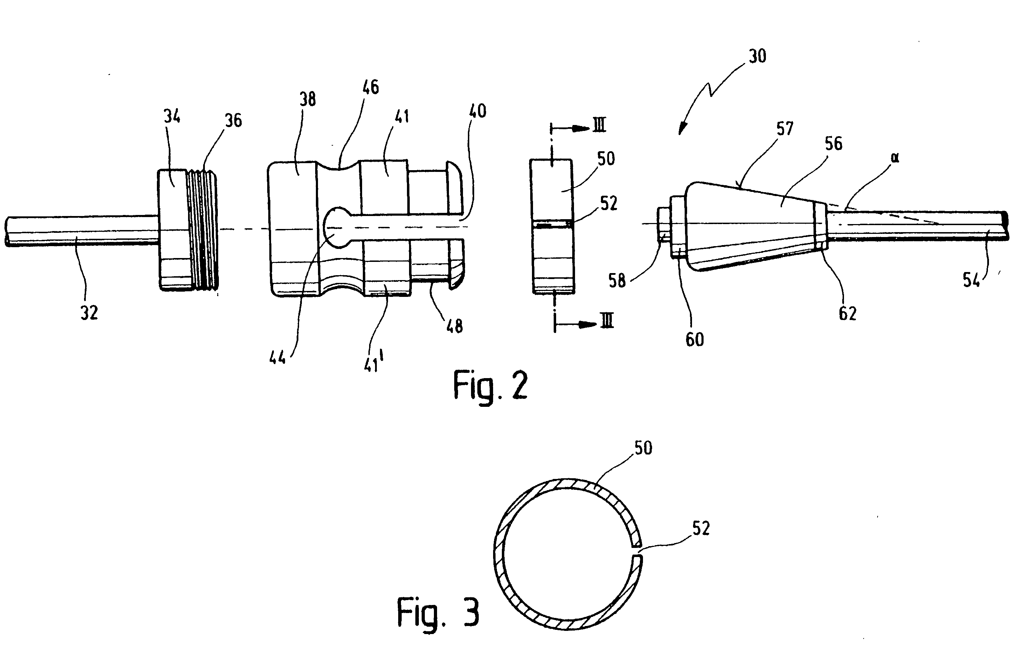

Wie in Fig. 1 und 2 dargestellt, steht die Vorrichtung 30 distal mit einem ersten Abschnitt 32 des stabförmigen Betätigungselementes 26 in Verbindung.

Dazu ist am proximalen Ende dieses Abschnittes 32 eine Schraube 34 mit einem Außengewinde 36 vorgesehen.

Die Schraube 34 ist dazu vorgesehen, in ein entsprechendes Innengewinde 37 am distalen Ende eines grob hohlzylinderförmigen Gehäuses 38 eingedreht zu werden.

In die Wand 42 des Gehäuses 38 sind, umfänglich gleichmäßig verteilt, vier sich axial erstreckende Nuten 40 eingeschnitten. Jede Nut 40 durchbricht vollständig die Wand 42 in radialer Richtung, öffnet nach proximal und erstreckt sich etwa über ca. zwei Drittel der axialen Länge des Gehäuses 38.

Jede Nut 40 endet in einer etwa kreisförmigen Aufweitung 44.

In umfänglicher Richtung gesehen liegen zwischen zwei benachbarten Nuten 40 dann entsprechende Segmente 41, 41' etc. der Wand 42 des Gehäuses 38.

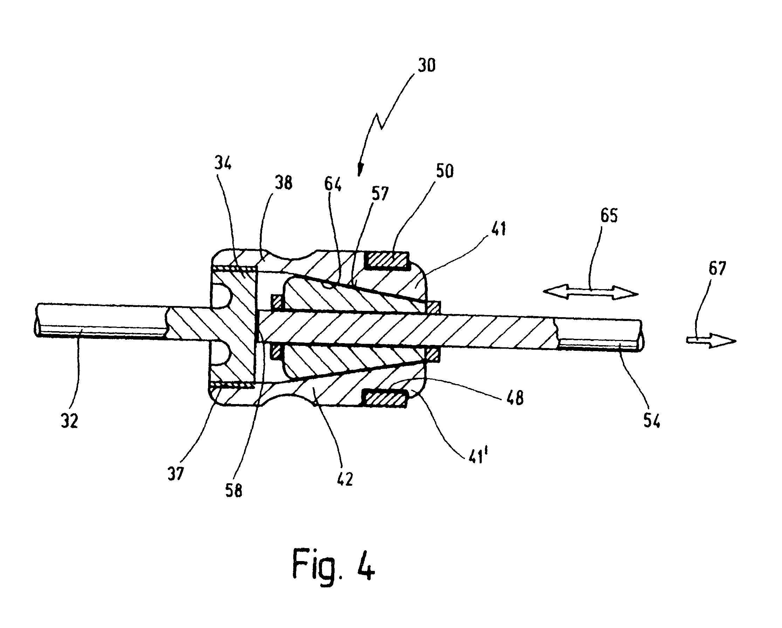

Im Bereich der Aufweitung 44 der Nut 40 ist an der Außenseite des Gehäuses 38 eine ringförmige Vertiefung 46 ausgespart, so daß in diesem Bereich die Dicke der Wand 42 reduziert ist, wie das insbesondere aus der Schnittdarstellung von Fig. 4 ersichtlich ist.

Im Bereich des proximalen Endes des Gehäuses 38 ist eine umfängliche Ringnut 48 eingeschnitten, deren Querschnitt etwa rechteckig ist.

Die Ringnut 48 dient dazu, einen Ring 50 aufzunehmen.

Aus der Schnittdarstellung von Fig. 3 ist ersichtlich, daß der Ring mit einem Schlitz 52 versehen ist.

Auf einen zweiten Abschnitt 54 des Betätigungselementes 26 ist ein Konus 56 montiert. Der Konus 56 weist eine konische Keilfläche 57 auf, die sich unter einem Keilwinkel α von ca. 10° von proximal nach distal gesehen anhebt. Der Konus 56 ist über zwei Befestigungsringe 60 und 62 auf dem Abschnitt 54 befestigt. Die Lage ist derart, daß distal vom Konus 56 ein Ende 58 des Abschnittes 54 vorsteht.

An der Innenseite der Wand 42 des Gehäuses 38 ist ein entsprechender Innenkonus 64 vorgesehen, wie das insbesondere aus Fig. 4 ersichtlich ist.

Die in Fig. 3 dargestellten Einzelteile werden so montiert, daß der geschlitzte Ring 52 über das proximale Ende des Gehäuses 38 geschoben wird, bis er in die Ringnut 48 einschnappt. Anschließend wird der zweite Abschnitt 54 mit darauf montiertem Konus 56 vom distalen Ende her in das Gehäuse 38 eingeschoben, bis dessen Keilfläche 57 am Innenkonus 64 anliegt.

Anschließend wird die Schraube 34 in das Innengewinde 37 eingedreht, bis die Stirnfläche der Schraube 34 an dem Ende 58 zum Liegen kommt, wie das in Fig. 4 dargestellt ist. In dieser Stellung liegt der Konus 56 an dem Innenkonus 64 an.

Der Zusammenbau kann, innerhalb eines bestimmten Kräftebereiches, hin und her bewegt werden, wie das in Fig. 4 durch einen Pfeil 65 angedeutet wird, ohne daß sich die Bauteile der Vorrichtung relativ zueinander verschieben.

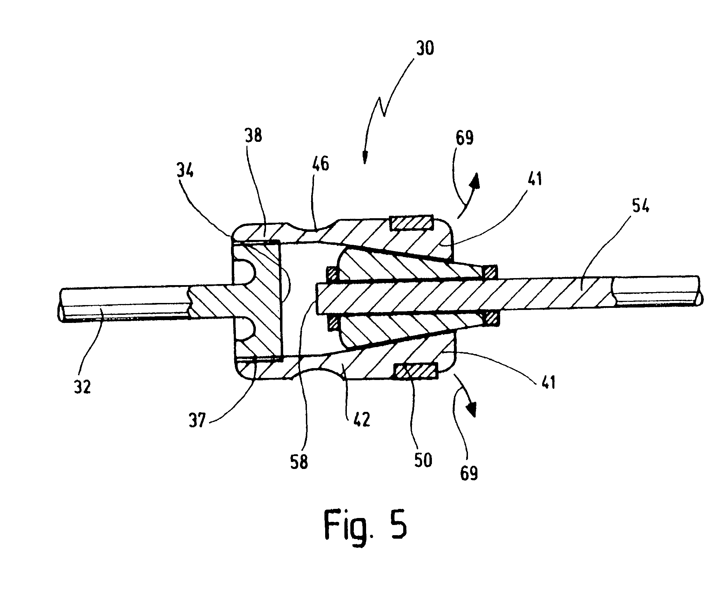

Wird beispielsweise die in Fig. 1 mit dem Pfeil 21 angedeutete Bewegung durchgeführt, bewegt sich der gesamte Zusammenbau aus den beiden Abschnitten 32 und 54 des Betätigungselementes 26, Gehäuse 38, Ring 50 und Konus 56 nach proximal, beispielsweise, bis die Maulteile 14 und 16 nur noch einen Winkel von 20° einschließen. Dies ist ein Winkel, bei dem beispielsweise eine Faßzange ein Gewebeteil oder ein chirurgisches Teil, sei es ein Mulltupfer oder eine Nadel, zwischen den Maulteilen 14, 16 hält. Ein weiteres Schließen der Maulteile über den Auslegungspunkt der Vorrichtung hinaus erfordert einen wesentlich höheren Kraftaufwand. Die Vorrichtung 30 sorgt nun dafür, daß diese Kraft nicht auf die Maulteile 14 und 16 übertragen wird, sondern daß diese Kraft definiert in Verformungsarbeit umgewandelt wird. Diese Umwandlung geschieht dadurch, daß, falls solche übermäßigen Kräfte an dem zweiten Abschnitt 54 angreifen, wie das in Fig. 4 durch einen Pfeil 67 dargestellt ist, sich der Konus 56 relativ zum Gehäuse 38 nach proximal bewegt und dabei die vier Segmente 41, 41' ... der geschlitzten Wand 42 radial spreizt, wie das in Fig. 5 durch die Pfeile 69 dargestellt ist.

Die filmscharnierartige Materialverdünnung im Bereich der Vertiefung 46 fördert die definierte Schwenkbewegung.

Der radiale Spreizweg ist durch den Ring 50 begrenzt, das heißt, der überwiegende Teil der übermäßigen Kraft wird zur Spreizung des Ringes 50 abgeleitet. Wie aus der Darstellung von Fig. 5 zu ersehen, hat dabei das Ende 58 des zweiten Abschnittes 54 vom ersten Abschnitt 32 abgehoben, wobei der Verschiebeweg in Fig. 5 aus darstellerischen Gründen übermäßig dargestellt ist, der Hub liegt im Bereich von 1 bis 3 mm.

Wirkt die übermäßige Kraft nicht mehr auf den zweiten Abschnitt 54, drückt der Ring 50 aufgrund seiner Elastizität das Gehäuse bzw. die Segmente 41, 41' ... wieder radial nach innen. Dadurch wird der Konus 56 wieder so weit nach distal verschoben, bis dessen Ende 58 an der Stirnfläche der Schraube 34 des ersten Abschnittes anstößt.

Aus der vorstehenden Beschreibung ergibt sich, daß die Federkennlinie der erfindungsgemäßen Vorrichtung 30 zum Begrenzen der Kraftübertragung durch drei wesentliche Parameter definiert wird, nämlich
- der Bauweise des Gehäuses 38, also der Auswahl des Materials, der Geometrie, der Anzahl der Nuten 36 und der Geometrie der ringförmigen Vertiefung 46;
- dem Konuswinkel α, dieser liegt im Bereich von ca. 8° bis 12°;
- der Konstruktion des Ringes 15, also Material, Form, also Dicke und Breite und Vorsehen eines Schlitzes 52 oder nicht. Bei metallischen Ringmaterialien wird immer ein Schlitz 52 vorgesehen sein, bei besonders elastischen Kunststoffmaterialien kann auch ein vollständig geschlossener Ring vorgesehen sein.

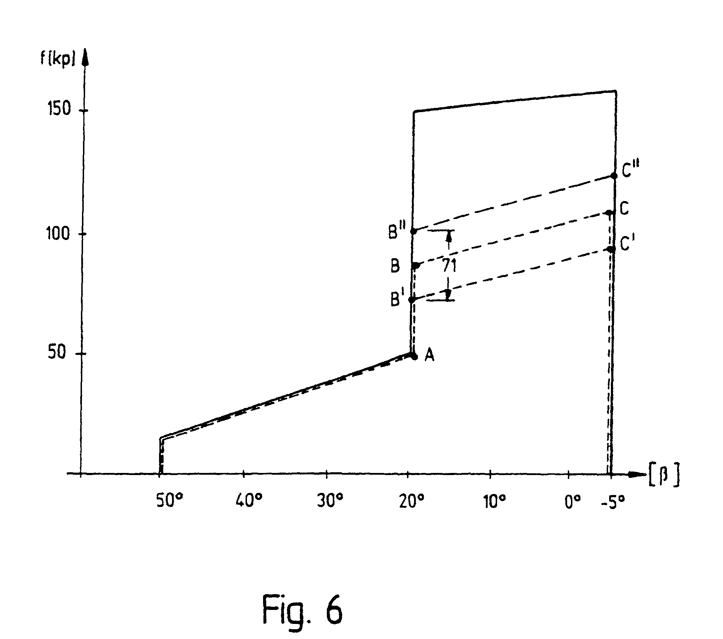

In Fig. 6 ist ein typisches Kraft-Weg-Diagramm der Maulteile einer Zange dargestellt, wobei der Gegenstand hier bei etwa 20° Öffnungswinkel fest gefaßt wird. Mit der durchgezogenen Linie ist das Diagramm einer Zange nach dem Stand der Technik ohne Kraftbegrenzung dargestellt ist.

Aus der Umhüllenden (Kraftenvelope) ist ersichtlich, daß ausgehend von einem maximalen Öffnungswinkel von 50° ein linearer geringer Anstieg bis zum gewünschten Schließwinkel von 20° erfolgt. Eine Erhöhung der einwirkenden Kraft bis etwa 150 kp führt dann zu einem weiteren Schließen, unter Verformung bis über den Schließwinkel 0° hinaus.

In gestrichelter Linie ist in dem Diagramm Fig. 6 die entsprechende Hüllkurve mit einer erfindungsgemäßen Vorrichtung 30 (dynamischer Kraftbegrenzer) dargestellt.

Der Punkt A stellt den Ausgangspunkt dar, nach dem die Maulteile 14 und 16 bis auf einen gewünschten Schließwinkel von etwa 20° eingestellt sind und der von der Zange aufgenommene Gegenstand fest gefaßt und mit zunehmender Haltekraft beaufschlagt wird.

Die Lage des Punktes A ist abhängig von den physikalischen Eigenschaften des von der Zange aufgenommenen Gegenstandes.

Der Punkt B stellt den Auslegungspunkt des Kraftbegrenzers dar, also den Punkt, an dem bei Überschreiten einer bestimmten Kraft, hier etwa 90 kp, die reversible Verformung beginnt. Höher einwirkende Kräfte werden nunmehr nicht mehr auf die Maulteile 14 und 16 abgeleitet, sondern zur Verformung der Bauelemente, Gehäuse 38 und Ring 50 herangezogen.

Durch einen Doppelpfeil 71 ist angedeutet, daß prinzipiell über die Variation der Bauelemente der Vorrichtung 30 ein gewisser Auslegungsbereich (hier B' bis B") frei wählbar ist.

Der Punkt C stellt den Grenzlastpunkt des Kraftbegrenzers dar. Der Punkt C verändert sich mit Variation des Auslegungspunktes B wie in Fig. 6 dargestellt.

Aus dem Verlauf der Umhüllenden (gestrichtelte Linie) bei Vorhandensein des Kraftbegrenzers im Vergleich zu der Umhüllenden (durchgezogenen Linie) ohne Kraftbegrenzer ist deutlich ersichtlich, daß die über den Auslegungspunkt aufgewandten Kräfte wirkungsvoll durch den Kraftbegrenzer umgesetzt werden und nicht auf die Maulteile übertragen werden.

Der Auslegungspunkt kann durch Verdrehen der Schraube 34 des ersten Abschnittes 32 fein eingestellt werden, das heißt, nachdem die Stirnfläche der Schraube 34 auf das Ende 58 getroffen ist, kann durch weiteres Eindrehen der Konus 56 schon etwas in Spreizrichtung verschoben werden und somit der Punkt B höher eingestellt werden.

Eine andere Einstellung des Auslegungspunktes B ist über die vorstehend beschriebenen Parameter möglich:
- Bauweise des Gehäuses 38
- Konuswinkel α
- Konstruktion des Ringes 15.

## Patentansprüche

1. Vorrichtung zum Begrenzen der Kraftübertragung von einem bewegbaren Betätigungselement (26) eines chirurgischen Instrumentes (10) auf die durch das Betätigungselement (26) bewegten arbeitenden Teile, insbesondere Maulteile (14, 16) von chirurgischen Zangen,
mit einem Betätigungselement (26), das ein Gehäuse (38) und zumindest eine relativ zum Gehäuse (38) bewegbare, dem Betätigungselement (26) zugehörige, quer zu dessen Bewegungsrichtung ansteigende Keilfläche (57) aufweist, **dadurch gekennzeichnet daß** das Gehäuse (38) durch Bewegen der Keilfläche (57) relativ zum Gehäuse (38) spreizbar ist, und mit einem das Gehäuse (38) in dessen Spreizbereich umfänglich umschließenden, durch den Spreizbereich elastisch spreizbaren Ring (50).

2. Vorrichtung nach Anspruch 1, **dadurch gekennzeichnet, daß** das Betätigungselement (26) axial längs eines Schaftes (12) des Instrumentes (10) verschiebbar ist und daß sich die Keilfläche (57) in axialer Richtung des Betätigungselementes (26) erstreckt.

3. Vorrichtung nach Anspruch 1 oder 2, **dadurch gekennzeichnet, daß** der Steigungswinkel α der Keilfläche (57) im Bereich von ca. 8° bis 12° liegt.

4. Vorrichtung nach einem der Ansprüche 1 bis 3, **dadurch gekennzeichnet, daß** die Keilfläche (57) als Außenfläche eines Konus (56) ausgebildet ist.

5. Vorrichtung nach einem der Ansprüche 1 bis 4, **dadurch gekennzeichnet, daß** das Gehäuse (38) sich in Bewegungsrichtung des Betätigungselementes (26) erstreckende, die Gehäusewand (42) durchbrechende Nuten (40) aufweist.

6. Vorrichtung nach Anspruch 5, **dadurch gekennzeichnet, daß** jede Nut (40) in einer abgerundeten Aufweitung (44) endet.

7. Vorrichtung nach Anspruch 5 oder 6, **dadurch gekennzeichnet, daß** das Gehäuse (38) vier gleichmäßig um dessen Umfang verteilte Nuten (40) aufweist.

8. Vorrichtung nach Anspruch 6, **dadurch gekennzeichnet, daß** das Gehäuse (38) im Bereich der Aufweitung (44) umfänglich eine rinnenförmige Vertiefung (46) aufweist, durch die die Dicke der Wand (42) des Gehäuses (38) reduziert ist.

9. Vorrichtung nach einem der Ansprüche 1 bis 8, **dadurch gekennzeichnet, daß** das Gehäuse (38) aus metallischem Material, insbesondere aus Medizinstahl, hergestellt ist.

10. Vorrichtung nach einem der Ansprüche 1 bis 8, **dadurch gekennzeichnet, daß** das Gehäuse (38) aus Kunststoffmaterial hergestellt ist.

11. Vorrichtung nach einem der Ansprüche 1 bis 10, **dadurch gekennzeichnet, daß** der spreizbare Ring (50) einen Schlitz (52) aufweist.

12. Vorrichtung nach einem der Ansprüche 1 bis 11, **dadurch gekennzeichnet, daß** der Ring (50) an unterschiedlichen axialen Positionen auf dem Gehäuse (38) anbringbar ist.

13. Vorrichtung nach einem der Ansprüche 1 bis 12, **dadurch gekennzeichnet, daß** der Ring (50) in einer Ringnut (48) am Gehäuse (38) aufgenommen ist.

14. Vorrichtung nach einem der Ansprüche 1 bis 13, **dadurch gekennzeichnet, daß** der Ring (50) aus metallischem Material, insbesondere aus Stahl hergestellt ist.

15. Vorrichtung nach einem der Ansprüche 1 bis 13, **dadurch gekennzeichnet, daß** der Ring (50) aus Kunststoffmaterial hergestellt ist.

16. Vorrichtung nach einem der Ansprüche 1 bis 15, **dadurch gekennzeichnet, daß** das Betätigungselement (26) einen ersten Abschnitt (32) aufweist, der mit dem Gehäuse (38) fest verbunden ist und ferner einen zweiten Abschnitt (54) aufweist, der vom ersten Abschnitt (32) getrennt ist, wobei der zweite Abschnitt (54) die Keilfläche (57) trägt, die innerhalb des Gehäuses (38) angeordnet ist.

17. Vorrichtung nach Anspruch 16, **dadurch gekennzeichnet, daß** in einer Normlast-Betriebsstellung der erste Abschnitt (32) am zweiten Abschnitt (54) anliegt und in einer Überlast-Betriebsstellung der zweite Abschnitt (54) sich vom ersten Abschnitt (32) gelöst hat.

18. Vorrichtung nach Anspruch 16 oder 17, **dadurch gekennzeichnet, daß** der erste Abschnitt (32) mit dem Gehäuse (38) über eine Schraubverbindung fest in Verbindung steht, wobei die Einschraubtiefe eine Einstellung eines Auslegungspunktes (B) der Vorrichtung (30) erlaubt.

19. Vorrichtung nach einem der Ansprüche 1 bis 15, **dadurch gekennzeichnet, daß** das Gehäuse (38) mit dem Griff (18) fest verbunden ist, wobei das Betätigungselement (26) die Keilfläche (57) trägt, die innerhalb des Gehäuses (38) angeordnet ist.

20. Chirurgisches Instrument mit einem Schaft (12), mit einem scherenartigen Griff (18) am proximalen Ende des Schaftes (12) und mit bewegbaren arbeitenden Maulteilen (14, 16) am distalen Ende des Schaftes (12), **dadurch gekennzeichnet, daß** es eine Vorrichtung (30) zum Begrenzen der Kraftübertragung nach einem der Ansprüche 1 bis 19 aufweist.

21. Chirurgisches Instrument nach Anspruch 20, **dadurch gekennzeichnet, daß** die Vorrichtung (30) im Bereich des Griffes (18) angeordnet ist.

## Claims

1. Apparatus for limiting the transfer of force from a movable actuation element (26) of a surgical instrument (10) to the working parts moved by the actuation element (26), in particular to mouth parts (14, 16) of surgical forceps, comprising an actuation element (26) having a housing (38) and at least one wedge surface (57) belonging to said actuation element (26) and rising perpendicular to its movement direction, **characterized in that** said housing (38) can be spread apart by movement of said wedge surface (57) relatively to said housing (38), and having an elastically spreadable ring (50) circumferentially surrounding the housing (38) in its spreading region.

2. Apparatus of claim 1, **characterized in that** the actuation element (26) is displaceable axially along a shaft (12) of the instrument (10); and **in that** the wedge surface (57) extends in an axial direction of the actuation element (26).

3. Apparatus of claims 1 or 2, **characterized in that** the angle of inclination α of the wedge surface (57) is in the range from approximately 8 degrees to 12 degrees.

4. Apparatus of anyone of claims 1 through 3, **characterized in that** the wedge surface (57) is configured as the outer surface of a cone (56).

5. Apparatus of anyone of claims 1 through 4, **characterized in that** the housing (38) has grooves (40) which extend in the movement direction of the actuation element (26) and pass through the housing wall (42).

6. Apparatus of claim 5, **characterized in that** each groove (40) ends in a rounded expansion (44).

7. Apparatus of claims 5 or 6, **characterized in that** the housing (38) has four grooves (40) distributed uniformly about its circumference.

8. Apparatus of claim 6, **characterized in that** the housing (38) has circumferentially, in the region of the expansion (44), a trough-like depression (46) which reduces the thickness of the wall (42) of the housing (38).

9. Apparatus of anyone of claims 1 through 8, **characterized in that** the housing (38) is made from metallic material, in particular from medical steel.

10. Apparatus of anyone of claims 1 through 8, **characterized in that** the housing (38) is made from plastic material.

11. Apparatus of anyone of claims 1 through 10, **characterized in that** the elastically spreadable ring (50) has a slot (52).

12. Apparatus of anyone of claims 1 through 11, **characterized in that** the ring (50) can be attached at different axial positions on the housing (38).

13. Apparatus of anyone of claims 1 through 12, **characterized in that** the ring (50) is received in an annular groove (48) of the housing (38).

14. Apparatus of anyone of claims 1 through 13, **characterized in that** the ring (50) is manufactured from metallic material, in particular from steel.

15. Apparatus of anyone of claims 1 through 13, **characterized in that** the ring (50) is made from plastic material.

16. Apparatus of anyone of claims 1 through 15, **characterized in that** the actuation element (26) has a first segment (32) which is immovably joined to the housing (38), and furthermore has a second segment (54) which is separate from the first segment (32), the second segment (54) carrying the wedge surface (57) which is arranged inside the housing (38).

17. Apparatus of claim 16, **characterized in that** in a normal load operating position the first segment (32) rests against the second segment (54), and in an overload operating position, the second segment (54) has detached from the first segment (32).

18. Apparatus of claims 16 or 17, **characterized in that** the first segment (32) is immovably joined to the housing (38) via a threaded connection, the depth to which it is threaded in allowing an adjustment of the design point (B) of the apparatus (30).

19. Apparatus of anyone of claims 1 through 15, **characterized in that** the housing (38) is immovably joined to the handle (18), the actuation element (26) carrying the wedge surface (57) which is arranged inside the housing (38).

20. Surgical instrument having a shaft (12), a scissor-like handle (18) at the proximal end of the shaft (12), and movable working mouth parts (14, 16) at a distal end of the shaft (12), **characterized in that** it has an apparatus (30) for limiting the transfer of force according to anyone of claims 1 through 19.

21. Surgical instrument of claim 20, **characterized in that** the apparatus (30) is arranged in the region of the handle (18).

## Revendications

1. Dispositif pour limiter la transmission des forces d'un élément d'actionnement (26) déplaçable d'un instrument chirurgical (10) aux parties de travail déplacées par l'élément d'actionnement (26), en particulier des parties de mâchoires (14, 16) de pinces chirurgicales, avec un élément d'actionnement (26) qui comporte un boîtier (38) et au moins une surface de coin (57) déplaçable par rapport au boîtier (38), faisant partie de l'élément d'actionnement (26), montant transversalement à sa direction de déplacement, **caractérisé en ce que** le boîtier (38) peut s'écarter, par déplacement de la surface de coin (57), par rapport au boîtier (38), et avec un anneau (50) enfermant périphériquement le boîtier (38) dans sa zone d'écartement, et écartable élastiquement sous l'effet de la zone d'écartement.

2. Dispositif selon la revendication 1, **caractérisé en ce que** l'élément d'actionnement (26) peut coulisser axialement le long d'une tige (12) de l'instrument (10) et **en ce que** la surface de coin (57) s'étend dans la direction axiale de l'élément d'actionnement (26).

3. Dispositif selon la revendication 1 ou 2, **caractérisé en ce que** l'angle d'inclinaison α de la surface de coin (57) se situe entre environ 8° et 12°.

4. Dispositif selon l'une des revendications 1 à 3, **caractérisé en ce que** la surface de coin (57) est réalisée en tant que surface extérieure d'un cône (56).

5. Dispositif selon l'une des revendications 1 à 4, **caractérisé en ce que** le boîtier (38) comporte des rainures (40) s'étendant dans la direction de déplacement de l'élément d'actionnement (26) et traversant la paroi de boîtier (42).

6. Dispositif selon la revendication 5, **caractérisé en ce que** chaque rainure (40) se termine dans un élargissement (44) arrondi.

7. Dispositif selon la revendication 5 ou 6, **caractérisé en ce que** le boîtier (38) comporte quatre rainures (40) réparties uniformément sur son pourtour.

8. Dispositif selon la revendication 6, **caractérisé en ce que** le boîtier (38) présente périphériquement, dans la zone de l'élargissement (44), un renfoncement (46) en forme de rigole qui réduit l'épaisseur de la paroi (42) du boîtier (38).

9. Dispositif selon l'une des revendications 1 à 8, **caractérisé en ce que** le boîtier (38) est fabriqué dans un matériau métallique, en particulier en acier médical.

10. Dispositif selon l'une des revendications 1 à 8, **caractérisé en ce que** le boîtier (38) est fabriqué dans une matière plastique.

11. Dispositif selon l'une des revendications 1 à 10, **caractérisé en ce que** l'anneau (50) écartable présente une fente (52).

12. Dispositif selon l'une des revendications 1 à 11, **caractérisé en ce que** l'anneau (50) peut être placé sur le boîtier (38) en différentes positions axiales.

13. Dispositif selon l'une des revendications 1 à 12, **caractérisé en ce que** l'anneau (50) est reçu dans une rainure annulaire (48) du boîtier (38).

14. Dispositif selon l'une des revendications 1 à 13, **caractérisé en ce que** l'anneau (50) est fabriqué dans un matériau métallique, en particulier en acier.

15. Dispositif selon l'une des revendications 1 à 13, **caractérisé en ce que** l'anneau (50) est fabriqué dans une matière plastique.

16. Dispositif selon l'une des revendications 1 à 15, **caractérisé en ce que** l'élément d'actionnement (26) comporte un premier tronçon (32) qui est solidaire du boîtier (38) et comporte en outre un deuxième tronçon (54) qui est séparé du premier tronçon (32), le deuxième tronçon (54) portant la surface de coin (57) qui est disposée à l'intérieur du boîtier (38).

17. Dispositif selon la revendication 16, **caractérisé en ce que** dans une position de fonctionnement sous charge normale, le premier tronçon (32) s'applique contre le deuxième tronçon (54) et dans une position de fonctionnement en surcharge, le deuxième tronçon (54) s'est séparé du premier tronçon (32).

18. Dispositif selon la revendication 16 ou 17, **caractérisé en ce que** le premier tronçon (32) est en liaison fixe avec le boîtier (38) par une liaison vissée, la profondeur du vissage permettant un réglage d'un point de dimensionnement (B) du dispositif (30).

19. Dispositif selon l'une des revendications 1 à 15, **caractérisé en ce que** le boîtier (38) est solidaire de la poignée (18), l'élément d'actionnement (26) portant la surface de coin (57) qui est disposée à l'intérieur du boîtier (38).

20. instrument chirurgical comportant une tige (12) avec une poignée (18) de type ciseaux à l'extrémité proximale de la tige (12) et avec des parties formant mâchoires de travail (14, 16) déplaçables à l'extrémité distale de la tige (12), **caractérisé en ce qu'**il comporte un dispositif (30) pour limiter la transmission des forces selon l'une des revendications 1 à 19.

21. instrument chirurgical selon la revendication 20, **caractérisé en ce que** le dispositif (30) est disposé dans la zone de la poignée (18).
